(19) Europäisches Patentamt
European Patent Office
Office européen des brevets

(11) Publication number: **0 096 931**
**B1**

(12) **EUROPEAN PATENT SPECIFICATION**

(45) Date of publication of patent specification: **25.03.87**

(51) Int. Cl.$^4$: **A 61 K 49/02**

(21) Application number: **83200827.0**

(22) Date of filing: **07.06.83**

(54) **Radiographic imaging agents.**

(30) Priority: **10.06.82 US 387135**

(43) Date of publication of application:
**28.12.83 Bulletin 83/52**

(45) Publication of the grant of the patent:
**25.03.87 Bulletin 87/13**

(84) Designated Contracting States:
**AT BE CH DE FR GB IT LI LU NL SE**

(56) References cited:
**EP-A-0 053 347**
**GB-A-1 489 330**
**GB-A-1 541 070**

**THE JOURNAL OF NUCLEAR MEDICINE, vol. 21, no. 4, April 1980, pages 366-370 A.J. TOFE et al.: "Gentisic acid: A new stabilizer for low tin skeletal imaging agents: Concise communication"**

(73) Proprietor: **Mallinckrodt, Inc. (a Delaware corporation)**
**675 McDonnell Boulevard P.O. Box 5840**
**St. Louis Missouri 63134 (US)**

(72) Inventor: **Benedict, James John**
**3916 North Cliff Lane**
**Cincinnati Ohio 45220 (US)**
Inventor: **Vanduzee, Barry Foster**
**1191 Highcliff Court**
**Cincinnati Ohio 45224 (US)**

(74) Representative: **BATCHELLOR, KIRK & EYLES et al**
**2 Pear Tree Court Farringdon Road**
**London EC1R 0DS (GB)**

(56) References cited:
**CHEMICAL ABSTRACTS, vol. 92, no. 18, 5th May 1980, page 406, no. 153120p, Columbus, Ohio, USA**
**A.J. TOFE et al., Radiopharm. Symposium, 2nd., 1979, pp. 637 et seq.**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 096 931 B1

**Description**

This invention relates to radiodiagnostic agents, including compositions which can be reconstituted to form radiodiagnostic agents particularly useful for skeletal imaging (bone scanning).

Scintigraphic skeletal imaging and similar radiographic techniques for visualizing other tissues are finding ever-increasing application in biological and medical research and in diagnostic procedures. Generally, scintigraphic procedures involve the preparation of radioactive agents which, upon introduction into a biological subject, become localized in specific organs, tissues, or skeletal structures that are under study. When so localized, traces, plots, or scintiphotos of the distribution of the radiographic materials can be made by various radiation detectors, e.g., traversing scanners and scintilation cameras. The distribution and corresponding relative intensity of the detected radioactive material not only indicates the position occupied by the tissue in which the radionuclide is localized, but also indicates the presence of aberrations, pathological conditions, and the like.

In general, depending on the type of radionuclide used and the organ of interest, a scintigraphic imaging agent as used in a hospital comprises a radionuclide, a carrier agent designed to target the specific organ, various auxiliary agents which affix the radionuclide to the carrier, water or other delivery vehicles suitable for injection into, or aspiration by, the patient, physiologic buffers and salts, and the like. The carrier attaches or complexes with the radionuclide and localizes the material in the location where the carrier naturally concentrates in a biologic subject.

Technetium-99m ($^{99m}Tc$) is a radionuclide which is widely used in tissue imaging agents. This radionuclide is conveniently available commercially in the oxidized pertechnetate form ($^{99m}TcO_4^-$, hereinafter "pertechnetate-Tc99m"). However, the technetium in pertechnetate has a valence state of +7 and, thus, will not complex with the most commonly used carriers for radionuclide tissue imaging. This problem is easily overcome by reducing the technetium to what is believed to be the +3, +4, and/or +5 valence state. Thus, technetium-labeled imaging agents are generally prepared by admixing pertechnetate-Tc99m isotonic saline solution with a technetium reductant (reducing agent) such as the stannous, ferrous, or chromous salt of sulfuric or hydrochloric acid, and the desired carrier agent for targeting the organ of interest. For example, organophosphonates are known as suitable carrier agents which target technetium radionuclide to bone tissue. U.S. Patent 3,983,227, Tofe and Francis, issued September 28, 1976, discloses the use of reducing salts with radioactive pertechnetate-Tc99m solutions and organophosphonate bone-seeking carriers to prepare skeletal imaging agents.

Technetium-containing scintigraphic imaging agents are known to be unstable in the presence of oxygen, primarily since oxidation of the reductant and/or the technetium destroys the reduced technetium/targeting carrier complex. Accordingly, such imaging agents are generally made oxygen-free by saturating the compositions with oxygen-free nitrogen gas or by preparing the agents in an oxygen-free atmosphere. However, such methods are not suited to commercial practice. Stabilization of imaging agents can also be achieved through chemical means. German Offenlegungsschrift 2,618,337, Tofe, published November 11, 1976, discloses the use of ascorbate stabilizers with technetium imaging agents. U.S Patent 4,232,000, Fawzi, issued November 4, 1980, discloses the use of gentisyl alcohol as a stabilizer for technetium imaging agents. Similarly, U.S. Patent 4,233,284, Fawzi, issued November 11, 1980, discloses the use of gentisic acid as a stabilizer.

Commercial products for use in skeletal imaging are generally provided in liquid or dry powder mixture imaging "kits" with vials containing phosphate or phosphonate bone seeking carriers. Such kits typically contain relatively substantial amounts of a reducing metal salt, such as stannous chloride. It has now been discovered that certain amino diphosphonates, when used in compositions containing low levels of stannous ions, unexpectedly provide excellent skeletal images.

The invention relates to an imaging kit, characterised in that it comprises:

(a) a carrier selected from compounds and mixtures of compounds having the formulae:

(I) 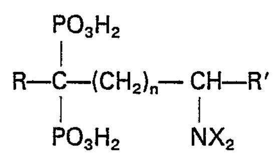 and (II) 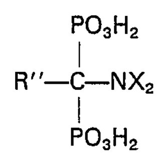

wherein n in an integer from 0 to 5; R is hydrogen, hydroxy, halogen, or amino; R' is hydrogen or lower alkyl containing from 1 to 5 carbon atoms; R" is hydrogen, halogen, lower alkyl, or aryl; X is hydrogen, lower alkyl, aryl, alkylaryl, acetyl, or haloaryl; and the pharmaceutically acceptable salts thereof; and

(b) an effective amount of reductant containing stannous tin;

wherein the molar ratio of said carrier to said stannous tin is greater than 50:1.

This invention is based on the discovery that a skeletal imaging kit containing aminodiphosphonates and low levels of a stannous reductant yields a skeletal imaging agent having excellent performance, manifested through fast blood clearance and high skeletal uptake of the technetium imaging agent, i.e., the bone-targeting carrier, complexed with technetium, is concentrated in bone tissue as compared to surrounding soft tissues in the body.

As used herein, the term "imaging" refers to all radiographic tissue imaging processes for which the instant compositions may be used, including (but not limited to) skeletal imaging. The term "imaging agent" herein refers to compositions useful for imaging, including (but not limited to) skeletal imaging, such compositions comprising the product of admixing pertechnetate-Tc99m, or other useful radioisotope, to an imaging kit comprising an aminodiphosphonate tissue-seeking carrier, stannous reductant, and, optionally, a stabilizer. Hence, the term "imaging kit", or "kit", refers to the imaging agent before addition of a solution of pertechnetate-Tc99m, or similar radionuclide.

The components of the composition herein, as well as methods of producing the composition, are described below. The quantity of these components incorporated into a preferred kit is enough to form multiple doses of imaging agent, as when reconstituted with a pertechnetate solution containing about $0.00371 \times 10^{-10}$ to $1.484 \times 10^{10}$ Bq (about 1 to 400 millicuries) of technetium-Tc99m. (The number of doses ultimately obtained from such a kit depends upon such factors as the weight of the dosed subject and the type of tissue to be imaged.) Generally, then, a preferred kit comprises:

(a) an amount of aminodiphosphonate carrier sufficient to target the technetium in a pertechnetate solution containing from $0.00371 \times 10^{10}$ to $1.484 \times 10^{10}$ Bq (1 to 400 mCi) of technetium-99m;

(b) an effective amount of stannous reductant sufficient to reduce the technetium in a pertechnetate solution containing from $0.00371 \times 10^{10}$ to $1.484 \times 10^{10}$ Bq (1 to 400 mCi) of technetium-99m, and

(c) an amount of stabilizer sufficient to prevent oxidation of the reductant and the reduced technetium-99m.

Components

The aminodiphosphonate bone-seeking carriers useful in the instant invention are selected from compounds and mixtures of compounds having the formulae:

(I) 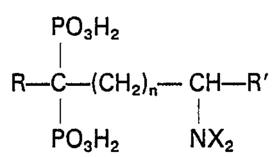 and (II) 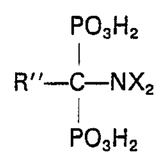

wherein n in an integer from 0 to 5; R is hydrogen, hydroxy, halogen, or amino; R' is hydrogen or lower alkyl (containing from 1 to 5 carbon atoms); R'' is hydrogen, halogen, lower alkyl (containing from 1 to 8 carbon atoms), or aryl; X is hydrogen, lower alkyl (containing from 1 to 8 carbon atoms), aryl, alkylaryl, acetyl, or haloaryl; and the pharmaceutically acceptable salts thereof.

Among the operable aminodiphosphonate carriers of formula (I) are: propane-1-hydroxy-3-amino-1,1-diphosphonic acid; ethane-1-hydroxy-2-amino-1,1-diphosphonic acid; butane-1-hydroxy-3-amino-1,1-diphosphonic acid; propane-1-hydroxy-2-amino-1,1-diphosphonic acid; propane-1-hydroxy-3-dimethyl-amino-1,1-diphosphonic acid; propane-1-hydroxy-3-diethylamino-1,1-diphosphonic acid; ethane-1-hydroxy-2-dimethylamino-1,1-diphosphonic acid; propane-1-hydroxy-1-dimethylamino-1,1-diphosphonic acid; and butane-1-hydroxy-3-dimethylamino-1,1-diphosphonic acid. Among the operable aminodiphosphonate carriers of formula (II) are: methaneaminodiphosphonic acid; methaneaminochlorodiphosphonic acid; methane-N-methylaminodiphosphonic acid; methane-N,N-dimethylaminodiphosphonic acid; methane-N-butylaminodiphosphonic acid; methane-N-phenylaminodiphosphonic acid; methane-N-naphthylaminodiphosphonic acid, methane-N-acetylaminodiphosphonic acid; methane-N-(chlorophenyl)-aminodiphosphonic acid; ethane-1-amino-1,1-diphosphonic acid; propane-1-amino-1,1-diphosphonic acid; butane-1-amino-1,1-diphosphonic acid; and ethane-1-amino-1,1-diphosphonic acid.

Compounds of these formulae are disclosed in U.S. Patent 3,983,227, Tofe, issued September 28, 1976 and U.S. Patent 4,054,598, Blum, et al., issued October 18, 1977.

Other operable aminodiphosphonate carriers are: methaneaminohydroxydiphosphonic acid, methanediaminodiphosphonic acid, methane-N-methylaminohydroxydiphosphonic acid, methane-N,N-dimethylaminohydroxydiphosphonic acid, methane-N-(2-hydroxyethyl)-aminodiphosphonic acid, and methane-N-(2-phenylethyl)-aminodiphosphonic acid.

Preferred aminodiphosphonate carriers include methaneaminodiphosphonic acid (AMDP), methane-N-methylaminodiphosphonic acid, methane-N,N-dimethylaminodiphosphonic acid, methaneaminohydroxydiphosphonic acid, propane-1-hydroxy-3-amino-1,1-diphosphonic acid, and ethane-1-hydroxy-2-amino-1,1-diphosphonic acid.

Any pharmaceutically-acceptable, water-soluble salt or hydrolyzable ester of these aminodiphosphonates are useful herein. The alkali metal and ammonium salts are preferred. Most preferred are the aminodiphosphonate free acids themselves, and the sodium salts thereof.

In order for these targeting carriers to be useful with technetium, commercially available technetium (as pertechnetate) must be reduced to form trivalent, tetravalent, and/or pentavalent technetium, which is then available to attach or complex with the targeting carrier. Reducing metal cations, such as stannous ion ($Sn^{+2}$) are known reductants for reducing the technetium in imaging compositions. The present invention incorporates one or more water-soluble, pharmaceutically-acceptable compounds which provide stannous ion when in solution, e.g., stannous chloride, stannous fluoride, and stannous sulfate, herein referred as

"reductant" or "stannous reductant". Stannous chloride ($SnCl_2$) is particularly preferred.

A sufficient amount of reductant must be included in an imaging kit to ensure complete reduction of the technetium-99m added in forming the imaging agent. This amount, herein "effective amount", is greater than or equal to (not less than) the stoichiometric amount to reduce all of the technetium in the pertechnetate to be added to the imaging kit, i.e., when the reductant is dissolved, there must be enough stannous ion in solution to reduce technetium (+7) to a lower valence state, facilitating complexation with an aminodiphosphonate carrier. Preferably, the effective amount is at least two times the molar amount of technetium to be added to the kit. The specific quantity of stannous reductant incorporated into an imaging kit encompassed by this invention may vary according to such factors as the molecular weight of the salt, the amount of pertechnetate to be added to the kit, the desired storage time of the agent made from the kit, the presence of oxidants in the agent, and the presence of antioxidant stabilizers, as discussed below. In the present invention, the effective amount of reductant is such that the ratio of moles of aminodiphosphonate to moles of stannous tin contained in the kit is not less than about 50:1. Preferably the effective amount is such that the ratio of moles is not less than about 55:1, more preferably 65:1, most preferably 75:1. The molar ratio should not exceed about 2,000:1. As used herein "stannous tin" refers to elemental $Sn^{+2}$ contained in the reductant compound.

Although optional, the imaging kits of this invention preferably contain a stabilizing amount of a stabilizer material to prevent or inhibit the oxidation of the reductant (e.g., oxidation of $Sn^{+2}$ to $Sn^{+4}$) during storage and/or to inhibit or reduce the reoxidation of reduced technetium-99m and/or to reduce the formation of technetium-labelled impurities which may form during use of the compositions. The presence of a stabilizer is especially preferred when the low levels of reductant encompassed by this invention are used in multi-dose kits. The stabilizers optionally used herein are characterized by their toxicological acceptability under the conditions of use, their ability to stabilize the product for a reasonable period of storage and/or under usage conditions, and by their substantial non-interference with the delivery of the technetium radionuclide to bone mineral.

Stabilizers that meet the foregoing requirements and that are quite suitable for intravenous injection include hydroquinone, gentisyl alcohol, gentisic acid, ascorbic acid, erythorbic acid, and their water-soluble salts and esters. Gentisic acid, ascorbic acid erythorbic acid, and their sodium salts are all known, commercially-available materials. The following documents describe useful stabilizers: U.S. Patent 4,232,200, Fawzi, issued November 4, 1980 (gentisyl alcohol); U.S. Patent 4,233,284, Fawzi, issued November 11, 1980 (gentisic acid); U.S. Patent 4,229,427, Whitehouse, issued October 21, 1980 (hydroquinone); and German Offenlegungsschrift 2,618,337, Tofe, published November 11, 1976 (ascorbic acid).

The sodium salt of ascorbic acid is a preferred stabilizer for use in a multi-dose dry-powder embodiment of this invention. Also preferred are the "reductate" stabilizers described in concurrently filed European Patent Application No. 83200826-2 (Publication No. 96930), "Stable Radiographic Imaging Agents", Fawzi, et al.

As is known in the literature, stabilizer materials such as ascorbic acid can chelate/complex with technetium and cause it to be deposited in uncalcified soft tissue. Since the user of a kit encompassed by the present invention will wish to avoid all unnecessary deposition of technetium in soft tissue, it will be appreciated that the amount of stabilizer material optionally included should not be so great as to overshadow the bone-directing effect of the diphosphonate carrier thereby interfering with the bone scan. Appropriate, non-interfering amounts of stabilizer materials for use in combination with the diphosphonates may vary according to the diphosphonate and/or stabilizer used. Guidelines for determining such amounts are known in the art.

## Composition and Methods

The composition of this invention comprises:

(1) an aminodiphosphonate carrier selected from the above defined group

(2) a stannous reductant, and optionally

(3) a stabilizer;

wherein the molar ratio of diphosphonate to stannous tin is greater than or equal to about 50:1, preferably greater than or equal to about 55:1, more preferably by greater than or equal to 65:1 most preferably greater than or equal to about 75:1. This "molar ratio", or "[DP/Sn]" herein, is the ratio of the number of moles of diphosphonate present in the composition to the number of moles of stannous tin present. A single unit-dose kit (that may be reconstituted to form an amount of imaging agent suitable for a single injection) for diphosphonates useful in the invention, containing no more than the maximum quantity of reductant encompassed by this invention, comprises at least from $2 \times 10^{-7}$ to $2 \times 10^{-6}$ moles of diphosphonate and $4 \times 10^{-9}$ moles stannous tin.

The imaging agents made with the kits of this invention are intended for intravenous injection into humans or lower animals. Accordingly, appropriate manufacturing and operating conditions are employed so as to provide suitably sterile, pyrogen-free compositions. Although not necessary to the practice of the present invention, it is preferable to use a pharmaceutically-acceptable extender or filler to dilute the reducing and diphosphonate salts in order to simplify metering the requisite small quantities of such salts. Sodium chloride and glucose are preferred; sodium chloride is especially preferred inasmuch as its

addition will assure that the resulting agent is at least isotonic even if the pertechnetate-Tc99m solution is hypotonic (as is the case when it must be diluted with sterile water to reduce its activity).

The compositions of the present invention can be prepared by simply dry mixing the technetium reductant and the diphosphonate carrier. The optional stabilizer can also be dry-blended into such mixtures, as can any additional, non-interfering agents such as sodium chloride. Such compositions are preferably placed in sterile vials fitted with a rubber septum, thereby facilitating mixing with a pertechnetate-Tc99m solution and convenient use in the hospital. The vials are preferably nitrogen-filled as an added protection against oxidation of the technetium reductant on storage.

In an alternate mode, the compositions herein can be provided as aqueous solutions in sterile, pyrogen-free water. Preferably, the water is deoxygenated and the composition is stored under nitrogen, thereby minimizing undesirable oxidation of the pertechnetate reductant on storage. Since the reductant is more prone to oxidize in solution than in the dry powder and freeze-dried composition forms, it is especially preferred that aqueous compositions contain a stabilizer.

In a preferred mode, the compositions herein can be provided in freeze-dried (lyophilized) form. Such compositions are prepared by co-dissolving the diphosphonate carrier and the technetium reductant in an aqueous solution, together with any desired optional stabilizers, and freeze-drying the composition using standard equipment. Preferably, sterile, deoxygenated water is used in processing and the product is stored under nitrogen. Although somewhat more complicated to manufacture than the dry mixture product, the freeze-dried product offers the advantage that water-insoluble particulate matter which might be present in the raw materials can be removed by filtration prior to the freeze drying step.

A preferred method of producing a lyophilized kit includes the steps of:

(1) preparing an aqueous solution of diphosphonate carrier, reductant, and optional stabilizer.

(2) adjusting the solution formed in step 1 to pH with a particular range; and

(3) lyophilizing the pH-adjusted solution.

The particular pH range employed in the process described above is dependent upon the presence and/or nature of stabilizer used in the kit composition. If there is no stabilizer used, or if gentisic acid, gentisyl alcohol, hydroquinone, or pharmaceutically acceptable salts thereof are used as a stabilizer, then the pH range employed is from 4.2 to 4.8, preferably about 4.5. If ascorbic acid, erythorbic acid, the pharmaceutically acceptable salts thereof, or reductate stabilizers are used, then the pH range employed is from 5.5 to 6.5, preferably about 6.0.

The preferred process of producing lyophilized kits, as described above, is described in concurrently filed European Patent Application Number 83200828.8 (Publication No. 96932), "Process for Making a Lyophilized Product For Use in Skeletal Imaging", Van Duzee (process incorporating diphosphonates with or without gentisic acid stabilizers) and in concurrently filed European Patent Application Number 83200829.6 (Publication No. 96933), "Process for Making a Lyophilized Product For Use in Skeletal Imaging", Van Duzee and Degenhardt (process incorporating diphosphonates with ascorbate or reductate stabilizers).

The compositions of this invention are dissolved with a pertechnetate-Tc99m isotonic solution from a commercial technetium source to yield an imaging agent suitable for intravenous injection. The stability of such imaging agents is ample under ordinary hospital conditions. Administration is preferably done within about eight hours after addition of the pertechnetate-Tc99m solution. Preferably, the concentration of reagents and technetium radionuclide is sufficient that about 1 milliliter of the solution is used in an adult of about 50—100 kg body weight. One milliliter of solution is preferably injected intravenously over a period of about 30 seconds. The total dosage of radionuclide for a sharp skeletal or myocardial infarct scan ranges from $0.0186 \times 10^{10}$ to $0.111 \times 10^{10}$ Bq (5 mCi to 30 mCi), preferably from $0.0371 \times 10^{10}$ to $0.0742 \times 10^{10}$ Bq (10 mCi to 20 mCi). See also U.S. Patent 4,234,562, Tofe et al., issued November 18, 1980; and U.S. Patent 4,247,534, Bevan, issued January 27, 1981.

The following non-limiting examples illustrate the composition, production, and use of the present invention.

## Example I

An imaging kit, encompassed by the present invention, was produced with the following ingredients:

| Component | Quantity in Bulk | Quantity in Kit |
|---|---|---|
| monosodium salt of AMDP | 300. mg | 3.0 mg |
| stannous chloride | 4.0 mg | 0.040 mg |
| sodium chloride | 3000. mg | 30.0 mg |

The AMDP and sodium chloride were dissolved in sterile, nitrogen-purged (deoxygenated) water. After dissolution of these components, the stannous chloride was dissolved in the solution. Sodium hydroxide

was added to adjust the pH to 4.5. Sterile, deoxygenated water was added to bring the solution volume to 100 ml.

One milliliter aliquots of the solution were placed in sterile, nitrogen purged vials. The vials were then freeze-dried (lyophilized) in a commercial lyophilizer, stoppered and sealed. The kit composition had a [DP/Sn] of about 67.

An imaging agent is prepared using this kit by adding about 5 ml of a pertechnetate-Tc99m physiological saline solution, with an activity of about $0.278 \times 10^{10}$ Bq (about 75 mCi), from a commercial technetium source. The vial is agitated until the kit components are dissolved. About 1 ml of the agent is slowly injected, over a period of about 30 seconds, into an adult human subject weighing about 75 kg. Excellent skeletal images are then obtained using a scintillation camera.

In the kit prepared above, methaneaminohydroxydiphosphonic acid, methane-N-methylaminodiphosphonic acid, methane-N,N-dimethylaminodiphosphonic acid, propane-1-hydroxy-3-amino-1,1-diphosphonic acid, ethane-1-hydroxy-2-amino-1,1-diphosphonic acid, and the monosodium salts thereof are, respectively, used instead of AMDP, with substantially similar results.

Example II

An imaging kit encompassed by the present invention, is produced with the following ingredients:

| | |
|---|---|
| monosodium salt of methane-N,N-dimethyldiphosphonic acid | 3.06 mg |
| stannous chloride | 0.037 mg |
| sodium chloride | 30.0 mg |
| gentisic acid | 0.80 mg |

The kit is prepared by blending the dry powder ingredients into sterile, oxygen-free vials. The vials are then sealed. The kit has a [DP/Sn] of about 65. Excellent skeletal images are obtained when this kit is used to prepare an imaging agent and the agent is injected, as in Example 1. In the foregoing kit, gentisyl alcohol, hydroquinone, and the sodium salts thereof are, respectively, added with substantially similar results.

Example III

An imaging kit encompassed by the present invention is produced with the following ingredients:

| Component | Quantity in Bulk | Quantity in Kit |
|---|---|---|
| monosodium salt of propane-1-hydroxy-3-amino-1,1-diphosphonic acid | 330.0 mg | 3.3 mg |
| stannous chloride | 4.9 mg | 0.049 mg |
| ascorbic acid | 71.0 mg | 0.71 g |
| sodium chloride | 600 mg | 6.0 mg |

Kits are made according to the process described in Example 1, except that the stabilizer is added and dissolved and the pH of the carrier/reductant/stabilizer solution is adjusted to a pH of about 6.0. The kits have a [DP/Sn] of about 50. When an imaging agent is made with this kit, and injected as in Example 1, excellent skeletal images are obtained.

In the kit prepared above, erythorbic acid, 6-bromo-6-deoxyascorbic acid, reductic acid, 5-methylreductic acid, nictinic acid and nicotinamide complexes thereof, and the sodium salts thereof are, respectively, used instead of ascorbic acid, with substantially similar results. Also, in the foregoing kit, stannous fluoride, stannous sulfate, stannous citrate, and stannous tartrate are, respectively, used instead of stannous chloride, in an amount sufficient to yield a [DP/Sn] of about 50 with substantially similar results. Further, in the foregoing kit, the relative amount of reductant used is varied (decreased) so that the kit has a [DP/Sn] of greater than 50, with substantially similar or better results.

Lyophilized kits were made, according to the process of Example 1, with the following compositions:

TABLE I

| Kit | Carrier (monosodium salt thereof) | Quantity of Carrier per Vial | Quantity of $SnCl_2$ per Vial | [DP/Sn] |
|---|---|---|---|---|
| A | AMDP | 3.01 mg | .02 mg | 134 |
| B | AMDP | 3.01 mg | .04 mg | 67 |
| C | AMDP | 3.01 mg | .08 mg | 34 |
| D | AMDP | 3.01 mg | .16 mg | 17 |
| E | AMDP | 3.01 mg | .24 mg | 11 |

(These kits contained no stabilizer).

Each kit vial was reconstituted by addition of 5 ml of a pertechnetate-Tc99m physiological saline solution with an average activity of approximately $.0888 \times 10^{10}$ Bq (24 mCi). A 50 microliter dose of each imaging agent thus formed was injected into a fasted rat of an average weight of about 200 g. One dose of an imaging agent was injected into each rat studied, with four rats injected for each kit made.

Scintiscans were taken of rats that were injected with agents made from each kit described in Table I, above. The rats were then sacrificed and blood, muscle, and bone tissue removed and placed in tared scintillation counting vials. The samples were weighed and radioassayed, along with a control sample of the original imaging agent made from each kit, using a gamma-scintillation spectrometer.

Table II, below, summarizes the distribution of the technetium-99m imaging agents in each rat's body, as a function of the [DP/Sn] of the imaging kit used to produce the agent. This distribution is a function of the carrier retention by various body tissues and is recorded in Table II as the relative bone to muscle retention ratio and the relative bone to blood retention ratio.

TABLE II

| Kit | Carrier | [DP/Sn] | Bone/Muscle | Bone/Blood |
|---|---|---|---|---|
| A | Aminodiphosphonomethane | 134 | 769 | 113 |
| B | Aminodiphosphonomethane | 62 | 899 | 125 |
| C | Aminodiphosphonomethane | 34 | 813 | 115 |
| D | Aminodiphosphonomethane | 17 | 562 | 78 |
| E | Aminodiphosphonomethane | 11 | 559 | 78 |

This data clearly demonstrates the effect on the distribution of technetium-99m imaging agent as a function of the [DP/Sn] of the kit used to produce the agent. In particular, at the higher values of [DP/Sn], the imaging agent is concentrated more heavily in bone tissue as compared to soft tissue and blood. Thus imaging kits comprising aminodiphosphonate carriers and stannous reductants, with a [DP/Sn] of at least 50, yield imaging agents with excellent imaging qualities, i.e., high bone/soft tissue and good blood clearance.

**Claims**

1. An imaging kit, characterised in that it comprises:

(a) a carrier selected from compounds and mixtures of compounds having the formulae:

(I) 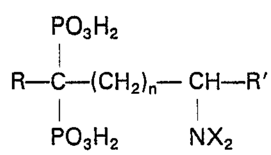 and (II) 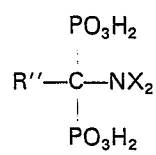

wherein n in an integer from 0 to 5; R is hydrogen, hydroxy, halogen, or amino; R′ is hydrogen or lower

alkyl containing from 1 to 5 carbon atoms; R'' is hydrogen, halogen, lower alkyl, or aryl; X is hydrogen, lower alkyl, aryl, alkylaryl, acetyl, or haloaryl; and the pharmaceutically acceptable salts thereof; and

(b) an effective amount of reductant containing stannous tin;

wherein the molar ratio of said carrier to said stannous tin is greater than 50:1.

2. An imaging kit, according to claim 1, characterised in that said molar ratio is at least 55:1.

3. An imaging kit, according to claim 1 or 2, characterised in that said molar ratio is at least 65:1.

4. An imaging kit, according to any of claims 1 to 3, characterised in that said molar ratio is at least 75:1.

5. An imaging kit, according to any one of claims 1 to 4, characterised in that said stannous reductant is stannous chloride.

6. An imaging kit, according to any of claims 1 to 5, characterised in that it further comprises an effective amount of a stabilizer selected from gentisic acid, gentisyl alcohol, ascorbic acid, erythorbic acid, and pharmaceutically acceptable salts and mixtures thereof.

7. An imaging kit, according to any of claims 1 to 6, characterised in that said carrier is selected from methaneaminodiphosphonic acid and the pharmaceutically acceptable salts and mixtures thereof.

8. An imaging kit, according to any of the preceding claims 1 to 6, characterised in that the carrier is selected from: methaneaminohydroxydiphosphonic acid, methanediaminodiphosphonic acid, methane-N-methylaminohydroxydiphosphonic acid, methane-N,N-dimethylaminohydroxydiphosphonic acid, methane-N-(2-hydroxyethyl)aminodiphosphonic acid, and methane-N-(2-phenylethyl)aminodiphosphonic acid.

9. An imaging kit, according to any one of claims 1 to 6, characterised in that said carrier is selected from propane-1-hydroxy-3-amino-1,1-diphosphonic acid and the pharmaceutically acceptable salts and mixtures thereof.

10. An imaging kit, according to any of claims 1 to 6, characterised in that said carrier is selected from methane-N,N-dimethylaminodiphosphonic acid and the pharmaceutically acceptable salts and mixtures thereof.

11. An imaging kit, according to any one of claims 1 to 5 and 7 to 10, characterised in that it further comprises a reductate stabilizer selected from 6-bromo-6-deoxyascorbic acid, 6-chloro-6-deoxyascorbic acid, reductic acid, 5-methylreductic acid, nicotinic acid and nicotinamide complexes thereof, and pharmaceutically acceptable salts and mixtures thereof.

**Patentansprüche**

1. Sichtbarmachungsausrüstung, dadurch gekennzeichnet, daß sie

(a) einen Träger, der unter Verbindungen und Gemischen von Verbindungen mit den Formeln:

(I) 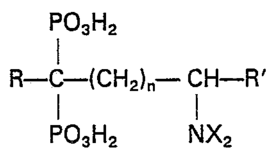 und (II) 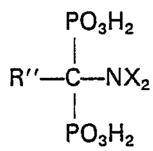 ,

worin n eine ganze Zahl von 0 bis 5, R Wasserstoff, Hydroxy, Halogen oder Amino, R' Wasserstoff oder niederes Alkyl mit 1 bis 5 Kohlenstoffatomen, R'' Wasserstoff, Halogen, niederes Alkyl oder Aryl, X Wasserstoff, niederes Alkyl, Aryl, Alkylaryl, Acetyl oder Halogenaryl sind, und deren pharmazeutisch geeigneten Salzen ausgewählt ist, und

(b) eine wirksame Menge Zinn(II) enthaltendes Reduktionsmittel

enthält, wobei das Molverhältnis des Trägers zu dem Zinn(II) größer als 50:1 ist.

2. Sichtbarmachungsausrüstung nach Anspruch 1, dadurch gekennzeichnet, daß das Molverhältnis wenigstens 55:1 ist.

3. Sichtbarmachungsausrüstung nach Anspruch 1 oder 2, dadurch gekennzeichnet, daß das Molverhältnis wenigstens 65:1 ist.

4. Sichtbarmachungsausrüstung nach einem der Ansprüche 1 bis 3, dadurch gekennzeichnet, daß das Molverhältnis wenigstens 75:1 ist.

5. Sichtbarmachungsausrüstung nach einem der Ansprüche 1 bis 4, dadurch gekennzeichnet, daß das Zinn(II)-Reduktionsmittel Zinn(II)-Chlorid ist.

6. Sichtbarmachungsausrüstung nach einem der Ansprüche 1 bis 5, dadurch gekennzeichnet, daß sie ferner eine wirksame Menge eines Stabilisators aufweist, der unter Gentisinsäure, Gentisylalkohol, Ascorbinsäure, Erythorbinsäure und deren pharmazeutisch geeigneten Salzen und Gemischen ausgewählt ist.

7. Sichtbarmachungsausrüstung nach einem der Ansprüche 1 bis 6, dadurch gekennzeichnet, daß der Träger unter Methanaminodiphosphonsäure und ihren pharmazeutisch geeigneten Salzen und Gemischen ausgewählt ist.

8. Sichtbarmachungsausrüstung nach einem der Ansprüche 1 bis 6, dadurch gekennzeichnet, daß der Träger unter Methanaminohydroxydiphosphonsäure, Methandiaminodiphosphonsäure, Methan-N-methylaminohydroxydiphosphonsäure, Methan-N,N-dimethylaminohydroxydiphosphonsäure, Methan-N-(2-hydroxyäthyl)-aminodiphosphonsäure und Methan-N-(2-phenyläthyl)-aminodiphosphonsäure ausgewählt ist.

9. Sichtbarmachungsausrüstung nach einem der Ansprüche 1 bis 6, dadurch gekennzeichnet, daß der Träger unter Propan-1-hydroxy-3-amino-1,1-diphosphonsäure und ihren pharmazeutisch geeigneten Salzen und Gemischen ausgewählt ist.

10. Sichtbarmachungsausrüstung nach einem der Ansprüche 1 bis 6, dadurch gekennzeichnet, daß der Träger unter Methan-N,N-dimethylaminodiphosphonsäure und ihren pharmazeutisch geeigneten Salzen und Gemischen ausgewählt ist.

11. Sichtbarmachungsausrüstung nach einem der Ansprüche 1 bis 5 und 7 bis 10, dadurch gekennzeichnet, daß sie ferner einen Reduktat-Stabilisator aufweist, der unter 6-Brom-6-desoxyascorbinsäure, 6-Chlor-6-desoxyascorbinsäure, Reduktinsäure, 5-Methylreduktinsäure, Nicotinsäure und ihren Nicotinamid-Komplexen und ihren pharmazeutisch geeigneten Salzen und Gemischen ausgewählt ist.

## Revendications

1. Kit de visualisation, caractérisé en ce qu'il comprend:

(a) un support choisi entre des composés et des mélanges de composés répondant aux formules:

(I) 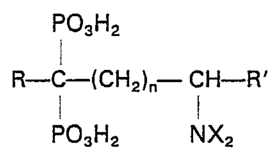 et (II) 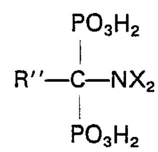

dans lesquelles *n* est un nombre entier de 0 à 5; R est l'hydrogène, un groupe hydroxy, un halogène ou un groupe amino; R' est l'hydrogène ou un groupe alkyle inférieur contenant 1 à 5 atomes de carbone; R'' est l'hydrogène, un halogène, un groupe alkyle inférieur ou aryle; X est l'hydrogène, un groupe alkyle inférieur, aryle, alkylaryle, acétyle ou halogénaryle; et leurs sels pharmaceutiquement acceptables; et

(b) une quantité efficace d'un réducteur contenant de l'étain stanneux;

le rapport molaire du support à l'étain stanneux étant supérieur à 50:1.

2. Kit de visualisation suivant la revendication 1, caractérisé en ce que ledit rapport molaire est d'au moins 55:1.

3. Kit de visualisation suivant la revendication 1 ou 2, caractérisé en ce que ledit rapport molaire est d'au moins 65:1.

4. Kit de visualisation suivant l'une quelconque des revendications 1 à 3, caractérisé en ce que ledit rapport molaire est d'au moins 75:1.

5. Kit de visualisation suivant l'une quelconque des revendications 1 à 4, caractérisé en ce que ledit réducteur stanneux est le chlorure stanneux.

6. Kit de visualisation suivant l'une quelconque des revendications 1 à 5, caractérisé en ce qu'il comprend en outre une quantité efficace d'un agent stabilisant choisi entre l'acide gentisique, l'alcool gentisylique, l'acide ascorbique, l'acide érythorbique et leurs sels pharmaceutiquement acceptables, et leurs mélanges.

7. Kit de visualisation suivant l'une quelconque des revendications 1 à 6, caractérisé en ce que le support est choisi entre l'acide méthane-aminodiphosphonique et ses sels pharmaceutiquement acceptables et leurs mélanges.

8. Kit de visualisation suivant l'une quelconque des revendications 1 à 6 précédentes, caractérisé en ce que le support est choisi entre: l'acide méthaneaminohydroxydiphosphonique, l'acide méthanediaminodiphosphonique, l'aciee méthane-N-méthylaminohydroxydiphosphonique, l'acide méthane-N,N-diméthylaminohydroxydiphosphonique, l'acide méthane-N-(2-hydroxyéthyl)aminodiphosphonique et l'acide méthane-N-(2-phényléthyl)aminodiphosphonique.

9. Kit de visualisation suivant l'une quelconque des revendications 1 à 6, caractérisé en ce que le support est choisi entre l'acide propane-1-hydroxy-3-amino-1,1-diphosphonique et ses sels pharmaceutiquement acceptables et leurs mélanges.

10. Kit de visualisation suivant l'une quelconque des revendications 1 à 6, caractérisé en ce que le support est choisi entre l'acide méthane-N,N-diméthylaminodiphosphonique et ses sels pharmaceutiquement acceptables et leurs mélanges.

11. Kit de visualisation suivant l'une quelconque des revendications 1 à 5 et 7 à 10, caractérisé en ce qu'il comprend en outre un agent de stabilisation du réducteur choisi entre l'acide 6-bromo-6-déoxyascorbique, l'acide 6-chloro-6-déoxyascorbique, l'acide réductique, l'acide 5-méthylréductique, l'acide nicotinique et leurs complexes de nicotinamide, et leurs sels pharmaceutiquement acceptables et leurs mélanges.